(19) Europäisches Patentamt
European Patent Office
Office européen des brevets

(11) Veröffentlichungsnummer: **0 019 760**
**B1**

(12) # EUROPÄISCHE PATENTSCHRIFT

(45) Veröffentlichungstag der Patentschrift: **23.06.82**

(51) Int. Cl.³: **A 01 N 43/56**, C 07 D 231/14, C 07 D 401/04, C 07 D 405/04, C 07 D 409/04, C 07 D 413/04

(21) Anmeldenummer: **80102483.7**

(22) Anmeldetag: **07.05.80**

(54) **3-Aryl-5-methyl-pyrazol-4-carbonsäureester, diese enthaltende Herbizide, Verfahren zu ihrer Herstellung und ihrer Anwendung.**

(30) Priorität: **23.05.79 DE 2920933**

(43) Veröffentlichungstag der Anmeldung: **10.12.80 Patentblatt 80/25**

(45) Bekanntmachung des Hinweises auf die Patenterteilung: **23.06.82 Patentblatt 82/25**

(84) Benannte Vertragsstaaten: **AT BE CH DE FR GB IT LI NL SE**

(56) Entgegenhaltungen: **US-A-4 116 673**

(73) Patentinhaber: **BASF Aktiengesellschaft, Carl-Bosch-Strasse 38, D-6700 Ludwigshafen (DE)**

(72) Erfinder: **Plath, Peter, Dr., Berner Weg 24, D-6700 Ludwigshafen (DE)**
Erfinder: **Rohr, Wolfgang, Dr., In der Dreispitz 13, D-6706 Wachenheim (DE)**
Erfinder: **Wuerzer, Bruno, Dr., Ruedigerstrasse 13, D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 760 B1

BUNDESDRUCKEREI BERLIN

# 3-Aryl-5-methyl-pyrazol-4-carbonsäureester, diese enthaltende Herbizide, Verfahren zu ihrer Herstellung und ihrer Anwendung

Die vorliegende Erfindung betrifft wertvolle Ester der 3-Aryl-5-methyl-pyrazol-4-carbonsäure und diese enthaltende Herbizide.

Es ist bekannt, 1-Äthyl-3-aryl-pyrazol-4-carbonsäureester als Herbizide zu verwenden (US-PS 4 116 673). Die dort angeführten biologischen Beispiele zeigen jedoch Aufwandmengen von 11,2 kg Wirkstoff je ha: In der allgemeinen Beschreibung zur Verwendbarkeit der Verbindungen werden 5,6 kg Wirkstoff oder mehr je Hektar als bevorzugt genannt. Speziell die Nachauflaufanwendung der bekannten Wirkstoffe läßt besondere herbizide Aktivitäten nicht erwarten. Gegen die Wirkstoffe unempfindliche Kulturpflanzen werden nicht erwähnt.

Es wurde nun gefunden, daß 3-Aryl-5-methyl-4-alkoxycarbonylpyrazole der Formel

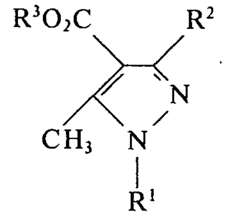

in welcher

- $R^1$ für Wasserstoff, Formyl, Acetyl, Propionyl, Chloracetyl, Dichloracetyl, Methoxyacetyl, Methoxycarbonyl oder für gegebenenfalls ein- oder mehrfach durch Halogen substituiertes Phenoxycarbonyl steht,
- $R^2$ für Phenyl, das gegebenenfalls ein- oder mehrfach durch Fluor, Chlor, Brom, Methyl, Methoxy, Cyan, Nitro, Trifluormethyl, Trifluormethoxy oder Methoxycarbonyl substituiert ist, oder für einen gegebenenfalls durch Methyl oder Chlor substituierten heterocyclischen aromatischen Fünf- oder Sechsring steht, der ein oder zwei Heteroatome X enthält, wobei X unabhängig voneinander Sauerstoff, Schwefel oder Stickstoff bedeutet, oder für $\alpha$-Naphthyl oder für $\beta$-Naphthyl steht,
- $R^3$ für Methyl, Äthyl oder Isopropyl steht, oder ein Salz dieser Pyrazolderivate,

gegenüber zahlreichen unerwünschten Pflanzen, insbesondere bei der Anwendung im Nachauflaufverfahren, eine überraschend starke herbizide Aktivität entfalten und gleichzeitig für verschiedene Kulturpflanzen ein akzeptables Maß an Verträglichkeit besitzen.

Die Verbindungen, in denen $R^3$ den Methylrest bedeutet, werden als solche beansprucht. Die Pyrazolderivate können als Salze mit üblichen anorganischen Säuren, wie Chlorwasserstoffsäure, Schwefelsäure, Ameisensäure, Methansulfonsäure, Trichloressigsäure oder p-Toluolsulfonsäure vorliegen.

Die Pyrazolderivate liegen meist als Isomere (für $R^1 = H$ als Tautomere) vor. So lange nicht erwähnt wird, daß lediglich eines der beiden Isomere vorliegt, soll im folgenden unter einer bestimmten Formel oder Bezeichnung stets zusätzlich auch das andere Isomere gemeint sein ohne daß es einer besonderen Erwähnung bedarf.

In der Formel der Pyrazolderivate steht $R^1$ beispielsweise für Wasserstoff, Formyl, Acetyl, Propionyl, Chloracetyl, Dichloracetyl, Methoxyacetyl, Methoxycarbonyl, Phenoxycarbonyl, p-Brom-phenoxycarbonyl, 2,3-Dichlorphenoxycarbonyl, 3,4-Dichlorphenoxycarbonyl oder 2,4-Dichlorphenoxycarbonyl.

$R^2$ steht beispielsweise für Phenyl, $\alpha$-Naphthyl, $\beta$-Naphthyl, 2-Methylphenyl, 3-Methylphenyl, 2-Fluorphenyl, 3-Fluorphenyl, 4-Fluorphenyl, 3-Chlorphenyl, 4-Chlorphenyl, 3-Bromphenyl, 3-Nitrophenyl, 3-Trifluormethyl-phenyl, 4-Trifluormethyl-phenyl, 3-Methoxyphenyl, 3-(1,1,1-Trifluormethyloxy)-phenyl, 4-(1,1,1-Trifluormethyloxy)-phenyl, 3-Cyanophenyl, 2,4-Dichlorphenyl; 2,5-Dichlorphenyl; 3,4-Dichlorphenyl; 3,5-Dichlorphenyl; 2,6-Difluorphenyl; 3,5-Dimethylphenyl; 3-Methoxycarbonylphenyl; 2-Methoxycarbonyl-phenyl; Thienyl-2, Thienyl-3; 4-Chlorthienyl-3; Furyl-2; Furyl-3; 4-Methyloxazolyl-5; Pyridyl-2; Pyridyl-3; Pyridyl-4; 2,6-Dichlor-pyridyl-4.

Die Herstellung der Wirkstoffe kann beispielsweise nach fünf Verfahren erfolgen.

1. Umsetzung von 2-Aroyl-3-methylamino-crotonsäurealkylestern mit Hydrazinhydrat (Ber. 42, 3912 [1909]).
2. Umsetzung von 2-Aroyl-acetessigestern mit Hydrazinhydrat (Ann. 279, 248 [1894]).
3. Eine weitere Darstellungsmöglichkeit der Pyrazolderivate besteht in der Oxidation von 3-Aryl-4-alkoxycarbonyl-5-methyl-pyrazolinen. Hierbei setzt man zunächst beispielsweise Benzalacetessigsäurealkylester mit Hydrazinhydrat um und oxidiert den erhaltenen Pyrazolin-carbonylsäureester. Beschrieben ist diese Darstellungsmethode allerdings nur für in 2-Stellung durch Phenyl substituierte Pyrazol-4-carbonsäureäthylester, die keine herbizide Wirkung besitzen (Ber. 59, 611 [1926]).

4. Schließlich kann man die Pyrazolderivate durch Addition von Diazoäthan an Phenylpropiolsäurealkylester erhalten.
Nach diesem Weg wurde erstmals eine der bevorzugten Verbindungen, nämlich das 3-Phenyl-4-methoxycarbonyl-5-methyl-pyrazol dargestellt (Comp. Rend. 273, Ser. C, 1772 [1971]).
5. Die Pyrazolderivate können schließlich auch erhalten werden, wenn man gegebenenfalls substituiertes Thiobenzhydrazid mit 2-Chloracetessigsäurealkylestern umsetzt (Arkiv för Kemi 8, 537 [1955]).

Bevorzugtes Herstellungsverfahren ist die unter 1. angegebene Methode.
Die nachstehende Vorschrift 1 soll zur näheren Erläuterung der Herstellung dienen.

Herstellung von 3-Phenyl-4-methoxycarbonyl-5-methylpyrazol

a) Herstellung von Methylamino-crotonsäuremethylester

Zu einer Lösung von 232 g (2 Mol) Acetessigsäuremethylester in 150 ml Wasser tropft man bei 5 bis 10°C 186 g (2,4 Mol) einer 40%igen wäßrigen Methylaminlösung. Anschließend läßt man 12 Stunden bei Raumtemperatur (20°C) rühren. Danach trennt man das Produkt durch Absaugen von der Lösung. Nach Waschen mit Eiswasser und Trocknen im Vakuum erhält man 212 g (82%) eines weißen Festoffes mit Fp. 62–63°C.

b) Herstellung von 2-Benzoyl-3-methylamino-buten-2-säure-methylester

Man löst 193,5 g (1,5 Mol) Methylaminocrotonsäuremethylester in 450 ml Toluol, fügt 166,7 g (1,65 Mol) Triäthylamin zu und tropft zu dieser Mischung unter Kühlung auf 0 bis 5°C 211 g (1,5 Mol) (=174 ml) Benzoylchlorid. Nach 12 Stunden Rühren bei 25°C wird vom ausgefallenen Triäthylaminhydrochlorid abgesaugt, anschließend wird die Toluollösung mit Wasser extrahiert. Nach Trocknen der Toluolphase mit $Na_2SO_4$ wird im Vakuum eingedampft und der erhaltene Feststoff aus Ligroin zu Toluol = 4 : 1 umkristallisiert. Man erhält 217 g (62%) eines weißen Feststoffs mit Fp. 75–77°C.

c) Herstellung von 3-Phenyl-4-methoxycarbonyl-5-methyl-pyrazol

Zu einer Lösung von 217 g (0,93 Mol) des unter b) erhaltenen Zwischenproduktes in 400 ml Eisessig tropft man bei 25 bis 30°C 48,8 g (0,98 Mol) Hydrazinhydrat und erhitzt die Mischung anschließend 1 Stunde auf 100°C. Durch Abkühlen auf 5°C und Zugabe von 600 ml Eiswasser fällt ein weißer Feststoff aus, der abgetrennt wird und mit $NaHCO_3$-Wasser nachgewaschen wird. Nach Trocknen im Vakuum erhält man 182 g (91%) des Pyrazolderivates mit Fp. 119–120°C.
In entsprechender Weise wurden die folgenden Substanzen erhalten:

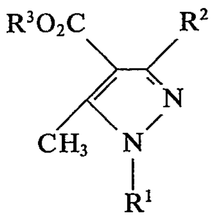

| Lfd. Nr. | $R^1$ | $R^2$ | $R^3$ | Fp (°C) |
|---|---|---|---|---|
| 1 | H | Phenyl | Methyl | 119–120 |
| 2 | H | Phenyl | Äthyl | 95–96 |
| 3 | H | Phenyl | iso-Propyl | 75–77 |
| 4 | H | $\alpha$-Naphthyl | Methyl | 142–143 |
| 5 | H | 2-Methylphenyl | Methyl | 99 |

Fortsetzung

| Lfd. Nr. | $R^1$ | $R^2$ | $R^3$ | Fp (°C) |
|---|---|---|---|---|
| 6 | H | 3-Methylphenyl | Methyl | 103–105 |
| 7 | H | 2-Fluorphenyl | Methyl | 95–96 |
| 8 | H | 3-Fluorphenyl | Methyl | 96 |
| 9 | H | 4-Fluorphenyl | Methyl | 110 |
| 10 | H | 3-Chlorphenyl | Methyl | 84–85 |
| 11 | H | 4-Chlorphenyl | Methyl | 116–118 |
| 12 | H | 3-Bromphenyl | Methyl | 63–65 |
| 13 | H | 3-Nitrophenyl | Methyl | 185–186 |
| 14 | H | 3–$CF_3$-phenyl | Methyl | 100–103 |
| 15 | H | 4-$CF_3$-phenyl | Methyl | 104–105 |
| 16 | H | 3-Methoxyphenyl | Methyl | 85–87 |
| 17 | H | 4-(1,1,1-Trifluormethyloxy)-phenyl | Methyl | 83–84 |
| 18 | H | 3-Cyano-phenyl | Methyl | 162–164 |
| 19 | H | 2,4-Dichlorphenyl | Methyl | 105 |
| 20 | H | 2,5-Dichlorphenyl | Methyl | 130 |
| 21 | H | 3,4-Dichlorphenyl | Methyl | 178–180 |
| 22 | H | 3,5-Dichlorphenyl | Methyl | 156–158 |
| 23 | H | 2,6-Difluorphenyl | Methyl | 114–117 |
| 24 | H | 3,5-Dimethylphenyl | Methyl | 175 |
| 25 | H | Thienyl-2 | Methyl | 122–124 |
| 26 | H | Thienyl-3 | Methyl | 135–137 |
| 27 | H | 4-Chlor-thienyl-3 | Methyl | 93–95 |
| 28 | H | Furyl-2 | Methyl | 116–119 |
| 29 | H | Furyl-3 | Methyl | 113–115 |
| 30 | H | 4-Methyl-oxazolyl-5 | Methyl | 133–134 |

Für den Fall, daß $R^1$ von Wasserstoff verschieden ist, wird die Herstellung der Verbindungen durch folgende Vorschriften erläutert:

Vorschrift 2

Herstellung von 1-Acetyl-3-phenyl-4-methoxycarbonyl-5-methyl-pyrazol

Man löst 17 g (0,08 Mol) des nach Vorschrift 1 hergestellten Pyrazolderivates in 40 ml Essigsäureanhydrid und erhitzt kurz zum Sieden. Nach 15 Minuten wird abkühlen gelassen und die

Mischung auf 150 g Eis gegossen. Nach 30 Minuten Rühren ist das Produkt vollständig auskristallisiert. Man saugt den weißen Feststoff ab und trocknet im Vakuum.

Ausbeute: 19,6 g (95%); Fp. 77–78° C.

Die Stellung des Acetylrestes wurde aus spektroskopischen Daten geschlossen (NMR-Spektrum). Sie ist nur durch eine Röntgenstrukturanalyse eindeutig zu beweisen.

Vorschrift 3

Herstellung von 1-Phenoxycarbonyl-3-phenyl-4-methoxycarbonyl-5-methyl-pyrazol

Zu einer Lösung von 17,3 g (0,08 Mol) des nach Vorschrift 1 erhaltenen Pyrazolderivates in 100 ml Tetrahydrofuran gibt man 8,9 g (0,088 Mol) Triäthylamin und tropft anschließend bei 15 bis 20° C 12,6 g (0,084 Mol) Chlorameisensäurephenylester zu. Nach 16stündigem Rühren bei 25° C wird vom ausgefallenen Triäthylaminhydrochlorid abgesaugt. Das Filtrat wird im Vakuum eingedampft und der zurückbleibende Feststoff mit Wasser durchgerührt. Das Produkt wird anschließend durch Absaugen von der Lösung abgetrennt und im Vakuum getrocknet.

Ausbeute 25 g (98%); Fp. 162–163° C.

In entsprechender Weise wurden die folgenden Verbindungen erhalten:

| Lfd. Nr. | $R^1$ | $R^2$ | $R^3$ | Fp (°C) |
|---|---|---|---|---|
| 31 | Acetyl | Phenyl | Methyl | 77–78 |
| 32 | Acetyl | Phenyl | Äthyl | 83–86 |
| 33 | Phenoxycarbonyl | Phenyl | Methyl | 162–163 |
| 34 | Acetyl | 3-Cyanophenyl | Methyl | 112–115 |
| 35 | Acetyl | 3-Nitrophenyl | Methyl | 135–137 |
| 36 | Acetyl | 3-Trifluormethyl-phenyl | Methyl | 67–68 |
| 37 | Acetyl | 4-Trifluormethyl-phenyl | Methyl | 50–53 |
| 38 | Acetyl | 2-Fluorphenyl | Methyl | 71–74 |
| 39 | Acetyl | 3-Fluorphenyl | Methyl | 75–78 |
| 40 | Acetyl | 4-Fluorphenyl | Methyl | 122–123 |
| 41 | Acetyl | 3-Chlorphenyl | Methyl | 92–94 |
| 42 | Acetyl | 3-Bromphenyl | Methyl | 79–80 |
| 43 | Acetyl | 4-Chlorphenyl | Methyl | 109–111 |
| 44 | Acetyl | 2,5-Dichlorphenyl | Methyl | 80–83 |
| 45 | Acetyl | 3,4-Dichlorphenyl | Methyl | 132–134 |
| 46 | Acetyl | 3,5-Dichlorphenyl | Methyl | 130–133 |
| 47 | Acetyl | Thienyl-2 | Methyl | 67–69 |
| 48 | H | 2,6-Dichlor-pyridyl-4 | Methyl | 172 |
| 49 | H | Pyridyl-2 | Methyl | 155–158 |

Fortsetzung

| Lfd. Nr. | $R^1$ | $R^2$ | $R^3$ | Fp (°C) |
|---|---|---|---|---|
| 50 | Formyl | Phenyl | Methyl | |
| 51 | Propionyl | Phenyl | Methyl | |
| 52 | Chloracetyl | Phenyl | Methyl | 112–115 |
| 53 | Dichloracetyl | Phenyl | Methyl | |
| 54 | Methoxyacetyl | Phenyl | Methyl | 115–117 |
| 55 | Methoxycarbonyl | Phenyl | Methyl | 84–86 |
| 56 | 2-Chlorphenoxycarbonyl | Phenyl | Methyl | 117–118 |
| 57 | 4-Chlorphenoxycarbonyl | Phenyl | Methyl | 147 |
| 58 | 2,4-Dichlorphenoxycarbonyl | Phenyl | Methyl | 115–117 |
| 59 | 2,5-Dichlorphenoxycarbonyl | Phenyl | Methyl | 110–114 |
| 60 | 3,4-Dichlorphenoxycarbonyl | Phenyl | Methyl | 130–132 |
| 61 | 3,5-Dichlorphenoxycarbonyl | Phenyl | Methyl | 125–128 |
| 62 | 2,3-Dichlorphenoxycarbonyl | Phenyl | Methyl | 140–143 |
| 63 | 4-Bromphenoxycarbonyl | Phenyl | Methyl | 154 |
| 64 | H | 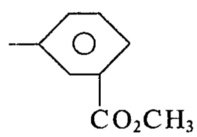 | Methyl | 110 |
| 65 | 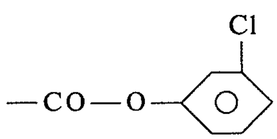 | Phenyl | Methyl | 121 |
| 66 | 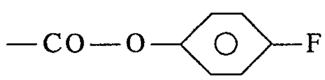 | Phenyl | Methyl | 145–147 |
| 67 | Acetyl | 3-Methylphenyl | Methyl | 55–58 |
| 68 | 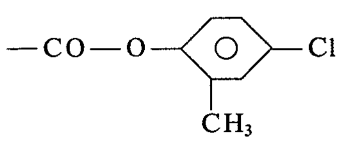 | 3-Cl-phenyl | Methyl | 136–139 |
| 69 | 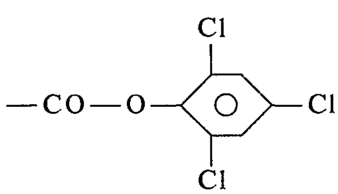 | 3-Cl-phenyl | Methyl | 111–113 |

Die Anwendung der Herbizide erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft- Dispergier- oder Emulgiermittel in Wasser homogenisiert werden. Es können aber auch aus wirksamer Substanz, Netz-, Haft-, Dispergier- oder Emulgiermittel und evtl. Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäure, Alkylarylsulfonate, Alkylsulfonate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenyl, -Octylphenol, -Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Die Herbizide enthalten beispielsweise 0,5 bis 95% (Gew.-%) Wirkstoff, vorzugsweise 1 bis 90%.

Die Aufwandmengen liegen je nach der Art des gewünschten Effektes zwischen 0,1 und 15 oder mehr, vorzugsweise jedoch zwischen 0,2 und 3,0 kg Wirkstoff pro Hektar.

Die 3-Aryl-5-methyl-pyrazol-4-carbonsäureester können unter sich und mit zahlreichen Vertretern anderer herbizider oder wachstumsregulierender Wirkstoffgruppen gemischt und gemeinsam ausgebracht werden. Beispielsweise kommen als Mischungskomponenten Diazine, Benzothiadiazinone, 2,6-Dinitroaniline, N-Phenyl-carbamate, Biscarbamate, Thiolcarbamate, Halogencarbonsäuren, Triazine, Amide, Harnstoffe, Diphenyläther, Triazinone, Uracile, Benzofuranderivate und andere in Betracht. Solche Kombinationen dienen zur Verbreiterung des Wirkungsspektrums und erzielen zuweilen synergistische Effekte.

Da die Herbizide vornehmlich Nachauflaufmittel sind, werden sie auch von Mischungspartnern, welche vorzugsweise über den Boden eine Wirkung zeigen, bestens ergänzt. Eine Reihe von Wirkstoffen, welche zusammen mit den Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon
5-Methylamino-4-chlor-2-m.trifluormethylphenyl-3(2H)-pyridazinon
5-Methylamino-4-chlor-2-m.$\alpha,\alpha,\beta,\beta$-tetrafluoräthoxyphenyl-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-m.trifluormethylphenyl-3(2H)-pyridazinon

5-Methoxy-4-chlor-2-m.trifluormethylphenyl-3(2H)-pyridazinon
5-Amino-4-brom-2-m.methylphenyl-3(2H)-pyridazinon
3-(1-Methyläthyl)-1H-2,1,3-benzothiadiazin-4(3H)-on-2,2-dioxid und Salze
3-(1-Methyläthyl)-8-chlor-1H-2,1,3-benzothiadiazin-4(3H)-on-2,2-dioxid und Salze
3-(1-Methyläthyl)-8-fluor-1H-2,1,3-benzothiadiazin-4(3H)-on-2,2-dioxid und Salze
3-(1-Methyläthyl)-8-methyl-1H-2,1,3-benzothiadiazin-4(3H)-on-2,2-dioxid und Salze
1-Methoxymethyl-3-(1-methyläthyl)-2,1,3-benzothiadiazin-4(3H)-on-2,2-dioxid
1-Methoxymethyl-8-chlor-3-(1-methyläthyl)-2,1,3-benzothiadiazin-4(3H)-on-2,2-dioxid
1-Methoxymethyl-8-fluor-3-(1-methyläthyl)-2,1,3-benzothiadiazin-4(3H)-on-2,2-dioxid
1-Cyan-8-chlor-3-(1-methyläthyl)-2,1,3-benzothiadiazin-4(3H)-on-2,2-dioxid
1-Cyan-8-fluor-3-(1-methyläthyl)-2,1,3-benzothiadiazin-4(3H)-on-2,2-dioxid
1-Cyan-8-methyl-3-(1-methyläthyl)-2,1,3-benzothiadiazin-4(3H)-on-2,2-dioxid
1-Cyan-3-(1-methyläthyl)-2,1,3-benzothiadiazin-4(3H)-on-2,2-dioxid
1-Azidomethyl-3-(1-methyläthyl)-2,1,3-benzothiadiazin-4(3H)-on-2,2-dioxid
3-(1-methyläthyl)-1H-(pyridino-[3,2-e]2,1,3-thiadiazin-(4)-on-2,2-dioxid
N-(1-Äthylpropyl)-2,6-dinitro-3,4-dimethylanilin
N-(1-Methyläthyl)-N-äthyl-2,6-dinitro-4-trifluormethyl-anilin
N-n.Propyl-N-β-chloräthyl-2,6-dinitro-4-trifluormethyl-anilin
N-n.Propyl-N-cyclopropylmethyl-2,6-dinitro-4-trifluor-methyl-anilin
N-Bis(n.propyl)-2,6-dinitro-3-amino-4-trifluormethylanilin
N-Bis(n.propyl)-2,6-dinitro-4-methyl-anilin
N-Bis(n.propyl)-2,6-dinitro-4-methylsulfonyl-anilin
N-Bis(n.propyl)-2,6-dinitro-4-aminosulfonyl-anilin
Bis(β-chloräthyl)-2,6-dinitro-4-methyl-anilin
N-Äthyl-N-(2-methylallyl)-2,6-dinitro-4-trifluormethyl-anilin
N-Methylcarbaminsäure-3,4-dichlorbenzylester
N-Methylcarbaminsäure-2,6-di(tert.butyl)-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Äthyl-2-(phenylcarbamoyl)-oxypropionsäureamid
3'-N-Isopropyl-carbamoyloxy-propionanilid
Äthyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-äthyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Äthyl-N-(3-N'-3-chlor-4-fluorphenylcarbamoyloxy)-phenyl)-carbamat
Äthyl-N-(3-N'-3,4difluorphenylcarbamoyloxy)-phenyl)-carbamat
Methyl-N-(3-(N'-3,4difluorphenylcarbamoyloxy)-phenyl)-carbamat
N-3-(4-Fluorphenoxycarbonylamino)-phenylcarbaminsäure-methylester
N-3-(2-Methylphenoxycarbonylamino)-phenylcarbaminsäure-äthylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester
N,N-Diäthyl-thiolcarbaminsäure-p-chlorbenzylester
N,N-Di n.propyl-thiolcarbaminsäure-äthylester
N,N-Di n.propyl-thiolcarbaminsäure-n.propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-äthyl-5-isoxazolyl-methylester
N,N-Di-sec.butyl-thiolcarbaminsäure-äthylester
N,N-Di-sec.butyl-thiolcarbaminsäure-benzylester
N-Äthyl-N-cyclohexyl-thiolcarbaminsäure-äthylester
N-Äthyl-N-bicyclo-[2,1,1]-heptyl-thiolcarbaminsäure-äthylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Äthyl-hexahydro-1-H-azepin-1-carbothiolat

S-Benzyl-3methylhexahydro-1-H-azepin-1-carbothiolat
S-Benzyl-2,3-dimethylhexahydro-1-H-azepin-1-carbothiolat
S-Äthyl-3methylhexahydro-1-H-azepin-1-carbothiolat
N-Äthyl-N-n.butyl-thiolcarbaminsäure-n.propylester
N,N-Dimethyl-dithiocarbaminsäure-2-chlorallylester
N-Methyl-dithiocarbaminsäure-Natrium
Trichloressigsäure-Na salz
α,α-Dichlorpropionsäure-Na salz
α,α-Dichlorbuttersäure-Na salz
α,α,β,β-Tetrafluorpropionsäure-Na salz
α-Methyl,α,β-dichlorpropionsäure-Na salz
α-Chlor-β-(4-chlorphenyl)-propionsäure-methylester
α,β-Dichlor-β-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureäthylester
2-[4-(4′-Chlorphenoxy)-phenoxy]-propionsäureisobutylester
2-[4-(2′,4′-Dichlorphenoxy)-phenoxy]-propionsäuremethylester
2-[4-(4′-Trifluormethylphenoxy)-phenoxy]-propionsäure-methylester
2-[4-(2′-Chlor-4′-trifluorphenoxy)-phenoxy]-propionsäure-Na salz
2-[4-(3′,5′-Dichlorpyridyl-2-oxy)-phenoxy]-propionsäure-Na salz
2-(N-Benzoyl-3,4-dichlorphenylamino)-propionsäureäthylester
2-(N-Benzoyl-3-chlor-4-fluorphenylamino)-propionsäure-methylester
2-(N-Benzoyl-3-chlor-4-fluorphenylamino)-propionsäureisopropylester
2-Chlor-4-äthylamino-6-isopropylamino-1,3,5-triazin
2-Chlor-4-äthylamino-6-(amino-2′-propionitril)-1,3,5-triazin
2-Chlor-4-äthylamino-6-2-methoxypropyl-2-amino-1,3,5-triazin
2-Chlor-4-äthylamino-6-butin-1-yl-2-amino-1,3,5-triazin
2-Chlor-4,6-bisäthylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-äthylamino-6-isopropylamino-1,3,5-triazin
2-Methylthio-4-äthylamino-6-tert.butylamino-1,3,5-triazin
2-Methylthio-4,6-bisäthylamino-1,3,5-triazin
2-Methylthio-4,6-bisisopropylamino-1,3,5-triazin
2-Methoxy-4-äthylamino-6-isopropylamino-1,3,5-triazin
2-Methoxy-4,6-bisäthylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion
3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil
2-Methyl-4-(3′-trifluormethylphenyl)-tetrahydro-1,2,4-oxadiazin-3,5-dion
2-Methyl-4-(4′-fluorphenyl)-tetrahydro-1,2,4-oxadiazin-3,5-dion
3-Amino-1,2,4-triazol
1-Allyloxy-1-(4-bromphenyl)-2-[1′,2′,4′-triazolyl-(1′)]-äthan (Salze)
[1-(1,2,4-Triazolyl-1′)]-[1(4′-chlorphenoxy)]-3,3-dimethylbutan-2-on
N,N-Diallylchloracetamid

N-Isopropyl-2-chloracetanilid
N-(Butin-1-yl-3)-2-chloracetanilid
2-Methyl-6-äthyl-N-(propargyl)-2-chloracetanilid
2-Methyl-6-äthyl-N-(äthoxymethyl)-2-chloracetanilid
2-Methyl-6-äthyl-N-(2-methoxy-1-methyläthyl)-2-chloracetanilid
2-Methyl-6-äthyl-N-(isopropoxycarbonyläthyl)-2-chloracetanilid
2-Methyl-6-äthyl-N-(4-methoxypyrazol-1-yl-methyl)-2-chlor-acetanilid
2-Methyl-6-äthyl-N-(pyrazol-1-yl-methyl)-2-chloracetanilid
2,6-Dimethyl-N-(pyrazon-1-yl-methyl)-2-chloracetanilid
2,6-Dimethyl-N-(4-methylpyrazol-1-yl-methyl)-2-chloracetatanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxalan-2-yl-methyl)-2-chloracetanilid
2,6-Dimethyl-N-(2-methoxyäthyl)-2-chloracetanilid
2,6-Dimethyl-N-(isobutoxymethyl)-2-chloracetanilid
2,6-Diäthyl-N-(methoxymethyl)-2-chloracetanilid
2,6-Diäthyl-N-(n.butoxymethyl)-2-chloracetanilid
2,6-Diäthyl-N-(äthoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diäthyl-N-(2-propoxyäthyl)-2-chloracetanilid
2-(2-Methyl-4-chlorphenoxy-N-methoxy-acetamid
2-(α-Naphthoxy)-N,N-diäthylpropionamid
2,2-Diphenyl-N,N-dimethylacetamid
α(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
N-2,4-Dimethyl-5-(trifluormethyl)-sulfonylamino-phenylacetamid
N-4-Methyl-5-(trifluormethyl)-sulfonylamino-phenylacetamid
2-Propionyl-amino-4-methyl-5-chlor-thiazol
O-(Methylsulfonyl)-glykolsäure-N-äthoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenamid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-Cyan-4-nitrophenylbenzaldoxim (Salze)
Pentachlorphenol-Natriumsalz
2,4-Dichlorphenyl-4′-nitrophenyläther
2,4,6-Trichlorphenyl-4′-nitrophenyläther
2-Fluor-4,6-dichlorphenyl-4′-nitrophenyläther
2-Chlor-4-trifluormethylphenyl-4′-nitrophenyläther
2,4′-Dinitro-4-trifluormethyl-diphenyläther
2,4-Dichlorphenyl-3′-methoxy-4′-nitro-phenyläther
2-Chlor-4-trifluormethylphenyl-3′-äthoxy-4′-nitro-phenyläther
2-Chlor-4-trifluormethylphenyl-3′-carboxy-4′-nitro-phenyläther (Salze)
2,4-Dichlorphenyl-3′-methoxycarbonyl-4′-nitro-phenyläther
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso.Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Äthoxy-2,3-dihydro-3,3-dimethyl-5-benzofuranyl-methan-sulfonat
2-Äthoxy-2,3-dihydro-3,3-dimethyl-5-benzofuranyl-dimethyl-aminosulfat
2-Äthoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)

2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-chlorphenyl)-1-benzoyl-3,3-dimethyl-harnstoff
1-(4-chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n.butyl-harnstoff
1-(4-i-Propylphenyl)-3,3-dimethyl-harnstoff
1-(3-Trifluormethylphenyl)-3,3-dimethyl-harnstoff
1-(α,α,β,β-Tetrafluoräthoxyphenyl)-3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooktyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Äthylen-2,2'-dipyridylium-dibromid
3-[1(N-Äthoxyamino)-propyliden]-6-äthyl-3,4-dihydro-2-H-pyran-2,4-dion
3-[1-(N-Allyloxyamino)-propyliden]-6-äthyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
α-Naphthoxyessigsäuremethylester

2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chloräthanphosphonsäure-2-chloräthylester
Ammonium-äthyl-carbamoyl-phosphonat
Di-n.butyl-1-n.butylamino-cyclohexyl-phosphonat
Trithiobutylphosphit
O,O-Diisopropyl-5-(2-benzosulfonylamino-äthyl)-phosphordithionat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chloräthyl)-trimethyl-ammoniumchlorid
(2-Methyl-4-phenylsulfonyl)-trifluormethansulfonanilid
1,1-Dimethyl-4,6-diisopropyl-5-indanyläthylketon
Natriumchlorat
Ammoniumrhodanid
Calciumcyanamid
2-Chlor-4-trifluormethylphenyl-3'-äthoxycarbonyl-4'-nitrophenyläther
1-(4-Benzyloxyphenyl)-3-methyl-3-methoxyharnstoff
2-[1-(2,5-Dimethylphenyl)-äthylsulfonyl]-pyridin-N-oxid.

Außerdem ist es nützlich, die Wirkstoffe allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- oder Spurenelementmängeln eingesetzt werden.

Zur Aktivierung der herbiziden Wirkung können Netz- und Haftmittel sowie nicht phytotoxische Öle und Ölkonzentrate zugesetzt werden.

Der Einfluß verschiedener Herbizide auf das Wachstum von unerwünschten und erwünschten Pflanzen wird in nachfolgenden Gewächshausversuchen demonstriert:

Als Kulturgefäße dienten Plastikblumentöpfe mit 300 $cm^3$ Inhalt und lehmiger Sand mit 1,5% Humus als Substrat. Die Samen der Testpflanzen entsprechend Tabelle 1 wurden nach Arten getrennt flach eingesät und für die Nachauflaufbehandlung bis zu einer Höhe von 3 bis 10 cm angezogen. Gewisse Testpflanzenarten wurden zunächst auch als Keimpflanzen in gesonderten Keimbehältern angezogen und danach in die oben beschriebenen Kulturgefäße umgepflanzt. Die Behandlung mit den chemischen Mitteln erfolgte dann einige Tage später nach dem Anwachsen. Die Wirkstoffe suspendierte oder emulgierte man in Wasser als Verteilungsmittel und spritzte sie mittels fein verteilender Düsen auf die Pflanzen. Die Aufstellung der Versuche erfolgte im Gewächshaus, wobei für wärmeliebende Arten heißere Bereiche (25 bis 40° C) und für solche gemäßigter Klimate 15 bis 30° C bevorzugt wurden. Die Versuchsperiode erstreckte sich über 3 bis 6 Wochen. Während dieser Zeit wurden die Pflanzen gepflegt und ihre Reaktion auf die einzelnen Behandlungen ausgewertet. Die folgenden Tabellen enthalten die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normalen Ablauf und 100 kein Aufgang der Pflanzen bzw. völlige Zerstörung zumindest der oberirdischen Sproßteile.

Die beigefügten Tabellen zeigen die selektive herbizide Wirkung der Herbizide. Die Applikation geschieht vorwiegend im Nachauflaufverfahren. Aber auch eine Vorauflaufwirkung ist vorhanden. Eine besondere Ausbringungstechnik besteht darin, daß die Wirkstoffe mit Hilfe der Spritzgeräte so gespritzt werden, daß die Blätter empfindlicher Kulturpflanzen nach Möglichkeit nicht getroffen werden, während die Wirkstoffe auf die darunterliegende Bodenfläche oder dort wachsende

unerwünschte Pflanzen gelangen (post-directed, lay-by). Dabei werden außer den in den Tabellen genannten unerwünschten Pflanzen auch die Flughaferarten (Avena fatua, Avena ludoviciana), Ackerfuchsschwanz (Alopecurus myosuroides) sowie zahlreiche andere unerwünschte Gräser und Kräuter erfaßt.

In Anbetracht der Vielseitigkeit der Applikationsmethoden können die Herbizide oder diese enthaltende Mischungen noch in einer weiteren großen Zahl von Kulturen zur Beseitung unerwünschten Pflanzenwuchses eingesetzt werden.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterübe | fooder beets |
| Beta vulgaris spp. esculenta | Rote Rübe | table beets, red beets |
| Brassica napus var. napus | Raps | rape |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor — Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus Carota | Möhre | carrots |
| Elaeis guineensis | Ölpalme | oil palms |

Fortsetzung

| Botanischer Name | Deutscher Name | Englischer Name |
|---|---|---|
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |

Fortsetzung

| Botanischer Name | Deutscher Name | Englischer Name |
|---|---|---|
| Albies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia fabe | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Tabelle 1

Liste der Testpflanzen

| Botanischer Name | Abkürzung in Tabelle | Deutscher Name | Englischer Name |
|---|---|---|---|
| Amaranthus retroflexus | Amar. retr. | Zurückgekrümmter Fuchsschwanz | redroot pigweed |
| Arachis hypogaea | Arachis hyp. | Erdnuß | peanuts (ground nuts) |
| Datura stramonium | Datura stram. | Gemeiner Stechapfel | jimsonweed |
| Echinochloa crus galli | Echinochl. c. g. | Hühnerhirse | barnyardgrass |
| Hordeum vulgare | Hordeum vulg. | Gerste | barley |
| Ipomoea spp. | | Prunkwindearten | morningglory |
| Lamium amplexicaule | Lamium amplexic. | Stengelumfassender Taubnessel | henbit |
| Nicandra physaloides | Nicandra phys. | Giftbeere | apple-of-Peru |
| Rumex optusifolium | Rumex obtus. | Sumpfblättiger Ampfer | duck |
| Sesbania exaltata | Sesbania exalt. | Turibaum | hemp sesbania (coffee weed) |
| Sorghum bicolor | | Mohrenhirse | sorghum |
| Sorghum halepense | Sorghum halep. | Sudangras | Johnsongrass |
| Sinapis alba | | Weißer Senf | white mustard |
| Triticum aestivum | Tritic. aest. | Weizen | wheat |
| Solanum nigrum | | Schwarzer Nachtschatten | black night shade |

Tabelle 2

Selektive Bekämpfung breitblättriger Unkräuter in Weizen bei Nachauflaufanwendung im Gewächshaus

| Wirkstoff Nr. | kg/ha | Testpflanzen und Schädigung % | | | |
|---|---|---|---|---|---|
| | | Tritic. aest. | Lamium amplexic. | Sinapis alba | Solanum nigrum |
| 5 | 0,5 | 10 | 80 | 95 | 100 |
| 22 | 0,25 | 0 | 100 | 95 | 100 |
| 28 | 0,5 | 0 | 90 | – | – |
| 6 | 0,25 | 0 | 100 | 95 | 100 |
| 8 | 0,25 | 10 | 100 | 95 | 100 |
| 12 | 0,25 | 10 | 100 | 90 | 100 |
| 7 | 0,25 | 0 | 100 | 90 | 100 |
| 9 | 0,25 | 10 | 100 | 90 | 100 |
| 16 | 0,25 | 10 | 100 | 90 | – |
| 18 | 0,5 | 0 | 100 | 85 | 100 |
| 42 | 0,25 | 0 | 100 | 95 | 100 |
| 35 | 1,0 | 0 | 100 | 95 | 100 |
| 38 | 0,5 | 0 | 100 | 90 | 100 |
| 39 | 0,25 | 10 | 100 | 95 | 100 |
| 40 | 0,25 | 0 | – | 90 | 100 |
| 46 | 0,25 | 0 | 100 | 95 | 100 |
| 41 | 0,25 | 0 | 100 | 95 | 100 |
| 47 | 0,25 | 0 | 100 | 90 | 100 |
| 48 | 1,0 | 10 | 100 | – | 100 |
| (US-PS 4 116 673) | 2,0 | 10 | 20 | 20 | 40 |

A = 1-Methyl-3-[3′-trifuormethyl-phenyl]-4-methoxycarbonyl-pyrazol

| | | | | | |
|---|---|---|---|---|---|
| (US-PS 4 116 673) | 2,0 | 0 | 40 | 20 | 30 |

B = 1-Methyl-3-phenyl-4-äthoxycarbonyl-pyrazol

0 = keine Schädigung
100 = Pflanzen abgestorben

Tabelle 3

Bekämpfung von breitblättrigen unerwünschten Pflanzen in Kultursorghum bei Nachauflaufanwendung im Gewächshaus

| Wirkstoff Nr. | kg/ha | Testpflanzen und Schädigung % | | | | |
|---|---|---|---|---|---|---|
| | | Sorghum bicolor | Amar. retr. | Datura stram. | Nicandra phys. | Sesbania exalt. |
| 6 | 0,25 | 10 | 80 | 98 | 100 | 100 |
| 14 | 1,0 | 10 | 100 | 100 | 100 | 100 |
| 33 | 1,0 | 5 | 70 | 100 | 100 | 100 |
| 34 | 2,0 | 10 | 100 | 100 | 100 | 100 |
| 8 | 0,5 | 0 | 100 | 100 | 100 | 98 |
| 41 | 0,5 | 0 | 100 | 100 | 100 | 100 |
| 47 | 1,0 | 5 | 100 | 100 | 100 | 100 |
| 1 | 0,5 | 5 | 70 | 100 | 100 | 100 |
| 7 | 0,5 | 0 | – | 98 | 100 | 100 |
| 42 | 0,5 | 10 | 100 | 100 | 100 | 100 |
| 40 | 1,0 | 10 | 100 | 100 | 100 | 100 |
| 46 | 1,0 | 10 | 100 | 100 | 100 | 100 |
| A | 2,0 | 5 | 0 | 10 | 20 | 90 |
| B | 2,0 | 15 | 0 | 30 | 40 | 10 |

0 = keine Schädigung
100 = Pflanzen abgestorben

Tabelle 4

Bekämpfung unerwünschten Pflanzenwuchses bei Erdnußpflanzen bei Nachauflaufanwendung im Gewächshaus

| Wirkstoff Nr. | kg/ha | Testpflanzen und Schädigung % | | | | |
|---|---|---|---|---|---|---|
| | | Arachis hyp. | Amar. retr. | Echinochl. c. g. | Ipomoea spp. | Sorghum halep. |
| 31 | 2,0 | 5 | 100 | 100 | 95 | 85 |
| 10 | 1,0 | 10 | 100 | 95 | 95 | 95 |
| 25 | 1,0 | 10 | 100 | 90 | 80 | 90 |
| 36 | 2,0 | 10 | 60 | 94 | 100 | 88 |
| 9 | 1,0 | 0 | 45 | 90 | 80 | 95 |
| 39 | 1,0 | 0 | 100 | 95 | 100 | 85 |
| 41 | 1,0 | 5 | 100 | 98 | 100 | 90 |
| 47 | 1,0 | 5 | 100 | 80 | 90 | 85 |
| 6 | 2,0 | 0 | 100 | 98 | 100 | 80 |
| 16 | 2,0 | 0 | 60 | 100 | 60 | 100 |
| 42 | 2,0 | 0 | 100 | 90 | 70 | 98 |
| A | 2,0 | 0 | 0 | 0 | 0 | 0 |
| (Us-PS (4116 673) | 2,0 | 0 | 0 | 0 | 0 | 0 |

C = 1-Methyl-3-[3'-trifluormethyl-phenyl]-4-äthoxycarbonyl-pyrazol

0 = keine Schädigung
100 = Pflanzen abgestorben

Tabelle 5

Herbizide Wirksamkeit bei Ipomoea spp. bei Nachauflaufanwendung im Gewächshaus

| Wirkstoff Nr. | kg/ha | Schädigung von Ipomoea spp. % |
|---|---|---|
| 15 | 3,0 | 100 |
| 24 | 3,0 | 100 |
| 32 | 3,0 | 100 |
| 37 | 3,0 | 100 |
| 43 | 3,0 | 100 |
| 44 | 3,0 | 100 |
| 45 | 3,0 | 100 |
| 26 | 3,0 | 90 |
| 29 | 3,0 | 100 |
| 52 | 3,0 | 100 |
| 54 | 3,0 | 100 |
| 55 | 3,0 | 100 |

Tabelle 6

Selektive Bekämpfung von unerwünschten Pflanzen in Getreide bei Nachauflaufanwendung im Gewächshaus

| Wirkstoff Nr. | kg/ha | Testpflanzen und Schädigung % | | | | |
|---|---|---|---|---|---|---|
| | | Hordeum vulg. | Tritic. aest. | Lamium amplex. | Rumex obtus. | Sinapis alba |
| 2 | 0,5 | 0 | 0 | 80 | 90 | 95 |
| B | 0,5 | 0 | 0 | 30 | 0 | 10 |

**Beispiel 1**

**Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.**

**Beispiel 2**

**20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an Salz der Dodecylbenzolsulfonsäure und 5 Gewichsteilen des Anlagerungsproduktes von 40 Mol Äthylenoxid an 1 Mol Ricinusöl besteht. Durch Ausgießen und feines Verteilen der Lösung in 10 000 Gewichtsteilen Wasser erhält man eine wäßrige Dispersion, die 0,02 Gew.-% des Wirkstoffs enthält.**

Beispiel 3

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel 4

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

Beispiel 5

20 Gewichtsteile des Wirkstoffs 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 6

3 Gewichtsteile der Verbindung 3 werden mit 97 Gewichtsteilen feinteiligem Kaolin innig vermischt. Man erhält auf diese Weise ein Stäubemittel, das 3 Gew.-% des Wirkstoffs enthält.

Beispiel 7

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 8

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

Beispiel 9

20 Teile des Wirkstoffs 2 werden mit 2 Teilen Calciumsalz der Dodecylbenzolsulfonsäure, 8 Teilen Fettalkokhol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LI, NL, SE**

1. Herbizid, enthaltend ein Pyrazolderivat der Formel

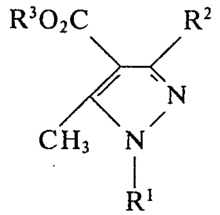

in welcher

$R^1$ für Wasserstoff, Formyl, Acetyl, Propionyl, Chloracetyl, Dichloracetyl, Methoxyacetyl, Methoxycarbonyl oder für gegebenenfalls ein- oder mehrfach durch Halogen substituiertes Phenoxycarbonyl steht,
$R^2$ für Phenyl, das gegebenenfalls ein- oder mehrfach durch Fluor, Chlor, Brom, Methyl, Methoxy, Cyan, Nitro, Trifluormethyl, Trifluormethoxy oder Methoxycarbonyl substituiert ist, oder für einen gegebenenfalls durch Methyl oder Chlor substituierten heterocyclischen aromatischen Fünf- oder Sechsring steht, der ein oder zwei Heteroatome X enthält, wobei X unabhängig voneinander Sauerstoff, Schwefel oder Stickstoff bedeutet, oder für α-Naphthyl oder für β-Naphthyl steht,
$R^3$ für Methyl, Äthyl oder Isopropyl steht,

oder ein Salz des Pyrazolderivats.

2. Herbizid, enthaltend einen festen oder flüssigen Trägerstoff und ein Pyrazolderivat wie im Anspruch 1 definiert.

3. Verfahren zur Herstellung eines Herbizids, dadurch gekennzeichnet, daß man einen festen oder flüssigen Trägerstoff vermischt mit einem Pyrazolderivat wie im Anspruch 1 definiert.

4. Verfahren zur Bekämpfung unerwünschten Pflanzenwuchses, dadurch gekennzeichnet, daß man den Boden oder die Pflanzen behandelt mit einem Pyrazolderivat wie im Anspruch 1 definiert.

5. Herbizid gemäß Anspruch 1, enthaltend ein Pyrazolderivat ausgewählt aus der Gruppe, bestehend aus

3-Phenyl-4-methoxycarbonyl-5-methyl-pyrazol,
3-Phenyl-4-ethoxycarbonyl-5-methyl-pyrazol,
1-Acetyl-3-m-nitrophenyl-4-methoxycarbonyl-5-methyl-pyrazol,
3-m-Methylphenyl-4-methoxycarbonyl-5-methyl-pyrazol,
3-m-Fluorphenyl-4-methoxycarbonyl-5-methyl-pyrazol,
3-o-Fluorphenyl-4-methoxycarbonyl-5-methyl-pyrazol,
1-Acetyl-3-m-chlorphenyl-4-methoxycarbonyl-5-methyl-pyrazol,
1-Acetyl-3-m-bromphenyl-4-methoxycarbonyl-5-methyl-pyrazol,
1-Acetyl-3-(3,5-dichlorphenyl)-4-methoxycarbonyl-5-methyl-pyrazol und
1-Acetyl-3-(thienyl-2)-4-methoxycarbonyl-5-methyl-pyrazol.

6. Pyrazolderivat der Formel

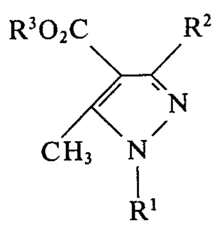

in welcher

$R^1$ für Wasserstoff, Formyl, Acetyl, Propionyl, Chloracetyl, Dichloracetyl, Methoxyacetyl, Methoxycarbonyl oder für gegebenenfalls ein- oder mehrfach durch Halogen substituiertes Phenoxycarbonyl steht,
$R^2$ für Phenyl, das ein- oder mehrfach durch Fluor, Chlor, Brom, Methyl, Methoxy, Cyan, Nitro, Trifluormethyl, Trifluormethoxy oder Methoxycarbonyl substituiert ist, oder für einen gegebenenfalls durch Methyl oder Chlor substituierten heterocyclischen aromatischen Fünf- oder

Sechsring steht, der ein oder zwei Heteroatome X enthält, wobei X unabhängig voneinander Sauerstoff, Schwefel oder Stickstoff bedeutet, oder für α-Naphthyl oder für β-Naphthyl steht,
$R^3$ für Methyl steht,

oder ein Salz des Pyrazolderivats.

7. Pyrazolderivat gemäß Anspruch 6 ausgewählt aus der Gruppe, bestehend aus

1-Acetyl-3-m-nitrophenyl-4-methoxycarbonyl-5-methyl-pyrazol,
3-m-Methylphenyl-4-methoxycarbonyl-5-methyl-pyrazol,
3-m-Fluorphenyl-4-methoxycarbonyl-5-methyl-pyrazol,
3-o-Fluorphenyl-4-methoxycarbonyl-5-methyl-pyrazol,
1-Acetyl-3-m-chlorphenyl-4-methoxycarbonyl-5-methyl-pyrazol,
1-Acetyl-3-m-bromphenyl-4-methoxycarbonyl-5-methyl-pyrazol,
1-Acetyl-3-(3,5-dichlorphenyl)-4-methoxycarbonyl-5-methyl-pyrazol und
1-Acetyl-3-(thienyl-2)-4-methoxycarbonyl-5-methyl-pyrazol.

**Patentansprüche für den Vertragsstaat: AT**

1. Herbizid, enthaltend ein Pyrazolderivat der Formel

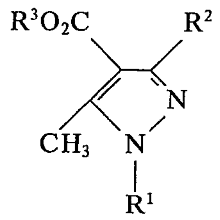

in welcher

$R^1$ für Wasserstoff, Formyl, Acetyl, Propionyl, Chloracetyl, Dichloracetyl, Methoxyacetyl, Methoxycarbonyl oder für gegebenenfalls ein- oder mehrfach durch Halogen substituiertes Phenoxycarbonyl steht,
$R^2$ für Phenyl, das gegebenenfalls ein- oder mehrfach durch Fluor, Chlor, Brom, Methyl, Methoxy, Cyan, Nitro, Trifluormethyl, Trifluormethoxy oder Methoxycarbonyl substituiert ist, oder für einen gegebenenfalls durch Methyl oder Chlor substituierten heterocyclischen aromatischen Fünf- oder Sechsring steht, der ein oder zwei Heteroatome X enthält, wobei X unabhängig voneinander Sauerstoff, Schwefel oder Stickstoff bedeutet, oder für α-Naphthyl oder für β-Naphthyl steht,
$R^3$ für Methyl, Äthyl oder Isopropyl steht,

oder ein Salz des Pyrazolderivats.

2. Herbizid, enthaltend einen festen oder flüssigen Trägerstoff und ein Pyrazolderivat wie im Anspruch 1 definiert.

3. Verfahren zur Herstellung eines Herbizids, dadurch gekennzeichnet, daß man einen festen oder flüssigen Trägerstoff vermischt mit einem Pyrazolderivat wie im Anspruch 1 definiert.

4. Verfahren zur Bekämpfung unerwünschten Pflanzenwuchses, dadurch gekennzeichnet, daß man den Boden oder die Pflanzen behandelt mit einem Pyrazolderivat wie im Anspruch 1 definiert.

5. Herbizid gemäß Anspruch 1, enthaltend ein Pyrazolderivat ausgewählt aus der Gruppe, bestehend aus

3-Phenyl-4-methoxycarbonyl-5-methyl-pyrazol,
3-Phenyl-4-ethoxycarbonyl-5-methyl-pyrazol,
1-Acetyl-3-m-nitrophenyl-4-methoxycarbonyl-5-methyl-pyrazol,
3-m-Methylphenyl-4-methoxycarbonyl-5-methyl-pyrazol,
3-m-Fluorphenyl-4-methoxycarbonyl-5-methyl-pyrazol,
3-o-Fluorphenyl-4-methoxycarbonyl-5-methyl-pyrazol,
1-Acetyl-3-m-chlorphenyl-4-methoxycarbonyl-5-methyl-pyrazol,
1-Acetyl-3-m-bromphenyl-4-methoxycarbonyl-5-methyl-pyrazol,
1-Acetyl-3-(3,5-dichlorphenyl)-4-methoxycarbonyl-5-methyl-pyrazol und
1-Acetyl-3-(thienyl-2)-4-methoxycarbonyl-5-methyl-pyrazol.

**Claims for the Contracting States: BE, CH, DE, FR, GB, IT, LI, NL, SE**

1. A herbicide containing a pyrazole derivative of the formula

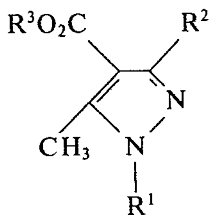

where $R^1$ denotes hydrogen, formyl, acetyl, propionyl, chloroacetyl, dichloroacetyl, methoxyacetyl, methoxycarbonyl, or phenoxycarbonyl which is unsubstituted or mono- or polysubstituted by halogen, $R^2$ denotes phenyl which is unsubstituted or mono- or polysubstituted by fluorine, chlorine, bromine, methyl, methoxy, cyano, nitro, trifluoromethyl, trifluoromethoxy or methoxycarbonyl, $R^2$ further denotes a 5- or 6-membered, heterocyclic aromatic ring which is unsubstituted or substituted by methyl or chlorine and which contains 1 or 2 hetero-atoms X, X being independently oxygen, sulfur or nitrogen, or $R^2$ denotes $\alpha$-naphthyl or $\beta$-naphthyl, and $R^3$ denotes methyl, ethyl or isopropyl, or a salt of the pyrazole derivative.

2. A herbicide containing a solid or liquid carrier and a pyrazole derivate as defined in claim 1.

3. A process for producing a herbicide, characterized in that a solid or liquid carrier is mixed with a pyrazole derivative as defined in claim 1.

4. A process for combating the growth of unwanted plants, characterized in that the soil or the plants are treated with a pyrazole derivative as defined in claim 1.

5. A herbicide as claimed in claim 1, containing a pyrazole derivative selected from the group consisting of

3-phenyl-4-methoxycarbonyl-5-methylpyrazole,
3-phenyl-4-ethoxycarbonyl-5-methylpyrazole,
1-acetyl-3-m-nitrophenyl-4-methoxycarbonyl-5-methylpyrazole,
3-m-methylphenyl-4-methoxycarbonyl-5-methylpyrazole,
3-m-fluorophenyl-4-methoxycarbonyl-5-methylpyrazole,
3-o-fluorophenyl-4-methoxycarbonyl-5-methylpyrazole,
1-acetyl-3-m-chlorophenyl-4-methoxycarbonyl-5-methylpyrazole,
1-acetyl-3-m-bromophenyl-4-methoxycarbonyl-5-methylpyrazole,
1-acetyl-3-(3,5-dichlorophenyl)-4-methoxycarbonyl-5-methylpyrazole and
1-acetyl-3-(thienyl-2)-4-methoxycarbonyl-5-methylpyrazole.

6. A pyrazole derivative of the formula

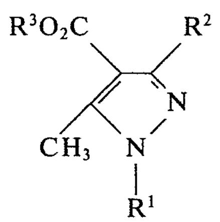

where $R^1$ denotes hydrogen, formyl, acetyl, propionyl, chloroacetyl, dichloroacetyl, methoxyacetyl, methoxycarbonyl, or phenoxycarbonyl which is unsubstituted or mono- or polysubstituted by halogen, $R^2$ denotes phenyl which is mono- or polysubstituted by fluorine, chlorine, bromine, methyl, methoxy, cyano, nitro, trifluoromethyl, trifluoromethoxy or methoxycarbonyl, $R^2$ further denotes a 5- or 6-membered, heterocyclic aromatic ring which is unsubstituted or substituted by methyl or chlorine and which contains 1 or 2 hetero-atoms X, X being independently oxygen, sulfur or nitrogen, or $R^2$ denotes $\alpha$-naphthyl or $\beta$-naphthyl, and $R^3$ denotes methyl, or a salt of the pyrazole derivative.

7. A pyrazole derivative as claimed in claim 6, selected from the group consisting of

1-acetyl-3-m-nitrophenyl-4-methoxycarbonyl-5-methylpyrazole,
3-m-methylphenyl-4-methoxycarbonyl-5-methylpyrazole,
3-m-fluorophenyl-4-methoxycarbonyl-5-methylpyrazole,
3-o-fluorophenyl-4-methoxycarbonyl-5-methylpyrazole,
1-acetyl-3-m-chlorophenyl-4-methoxycarbonyl-5-methylpyrazole,

1-acetyl-3-m-bromophenyl-4-methoxycarbonyl-5-methylpyrazole,
1-acetyl-3-(3,5-dichlorophenyl)-4-methoxycarbonyl-5-methylpyrazole and
1-acetyl-3-(thienyl-2)-4-methoxycarbonyl-5-methylpyrazole.

**Claims for the Contracting State: AT**

1. A herbicide containing a pyrazole derivative of the formula

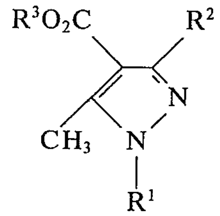

where $R^1$ denotes hydrogen, formyl, acetyl, propionyl, chloroacetyl, dichloroacetyl, methoxyacetyl, methoxycarbonyl, or phenoxycarbonyl which is unsubstituted or mono- or polysubstituted by halogen, $R^2$ denotes phenyl which is unsubstituted or mono- or polysubstituted by fluorine, chlorine, bromine, methyl, methoxy, cyano, nitro, trifluoromethyl, trifluoromethoxy or methoxycarbonyl, $R^2$ further denotes a 5- or 6-membered, heterocyclic aromatic ring which is unsubstituted or substituted by methyl or chlorine and which contains 1 or 2 hetero-atoms X, X being independently oxygen, sulfur or nitrogen, or $R^2$ denotes $\alpha$-naphthyl or $\beta$-naphthyl, and $R^3$ denotes methyl, ethyl or isopropyl, or a salt of the pyrazole derivative.

2. A herbicide containing a solid or liquid carrier and a pyrazole derivative as defined in claim 1.

3. A process for producing a herbicide, characterized in that a solid or liquid carrier is mixed with a pyrazole derivative as defined in claim 1.

4. A process for combating the growth of unwanted plants, characterized in that the soil or the plants are treated with a pyrazole derivative as defined in claim 1.

5. A herbicide as claimed in claim 1, containing a pyrazole derivative selected from the group consisting of

3-phenyl-4-methoxycarbonyl-5-methylpyrazole,
3-phenyl-4-ethoxycarbonyl-5-methylpyrazole,
1-acetyl-3-m-nitrophenyl-4-methoxycarbonyl-5-methylpyrazole,
3-m-methylphenyl-4-methoxycarbonyl-5-methylpyrazole,
3-m-fluorophenyl-4-methoxycarbonyl-5-methylpyrazole,
3-o-fluorophenyl-4-methoxycarbonyl-5-methylpyrazole,
1-acetyl-3-m-chlorophenyl-4-methoxycarbonyl-5-methylpyrazole,
1-acetyl-3-m-bromophenyl-4-methoxycarbonyl-5-methylpyrazole,
1-acetyl-3-(3,5-dichlorophenyl)-4-methoxycarbonyl-5-methylpyrazole and
1-acetyl-3-(thienyl-2)-4-methoxycarbonyl-5-methylpyrazole.

**Revendications pour les Etats Contractants: BE, CH, DE, FR, GB, IT, LI, NL, SE**

1. Herbicide contenant un dérivé pyrazolique de formule:

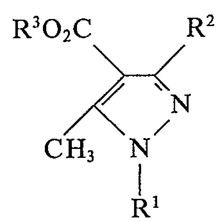

dans laquelle

$R^1$ représente hydrogène, formyle, acétyle, propionyle, chloracétyle, dichloracétyle, méthoxyacétyle, méthoxycarbonyle ou phénoxycarbonyle éventuellement substitué une ou plusieurs fois par un halogène,

$R^2$ représente phényle, qui est substitué éventuellement une ou plusieurs fois par fluor, chlore, brome, méthyle, méthoxy, cyano, nitro, trifluorométhyle, trifluorométhoxy ou méthoxycarbonyle, ou un noyau aromatique hétérocyclique penta- ou hexagonal, éventuellement substitué par

méthyle ou chlore, et contenant un ou deux hétéroatomes X, X signifiant, indépendamment l'un de l'autre oxygène, soufre ou azote, ou représentant $\alpha$-naphtyle ou $\beta$-naphtyle,
$R^3$ représente méthyle, éthyle ou isopropyle ou un sel du dérivé pyrazolique.

2. Herbicide contenant un véhicule solide ou liquide et un dérivé pyrazolique tel que défini à la revendication 1.

3. Procédé de préparation d'un herbicide, caractérisé par le fait qu'on mélange un véhicule solide ou liquide avec un dérivé pyrazolique tel que défini à la revendication 1.

4. Procédé de lutte contre la croissance indésirable de plantes, caractérisé par le fait qu'on traite le sol ou les plantes avec un dérivé pyrazolique tel que défini à la revendication 1.

5. Herbicide selon la revendication 1, contenant un dérivé pyrazolique choisi dans le groupe constitué de:

3-phényl-4-méthoxycarbonyl-5-méthyl-pyrazole,
3-phényl-4-éthoxycarbonyl-5-méthyl-pyrazole,
1-acétyl-3-m-nitrophényl-4-méthoxycarbonyl-5-méthyl-pyrazole,
3-m-méthylphényl-4-méthoxycarbonyl-5-méthyl-pyrazole,
3-m-fluorophényl-4-méthoxycarbonyl-5-méthyl-pyrazole,
3-o-fluorophényl-4-méthoxycarbonyl-5-méthyl-pyrazole,
1-acétyl-3-m-chlorophényl-4-méthoxycarbonyl-5-méthyl-pyrazole,
1-acétyl-3-m-bromophényl-4-méthoxycarbonyl-5-méthyl-pyrazole,
1-acétyl-3-(3,5-dichlorophényl)-4-méthoxycarbonyl-5-méthyl-pyrazole et
1-acétyl-3-(thiényl-2)-4-méthoxycarbonyl-5-méthylpyrazole.

6. Dérivé pyrazolique de formule:

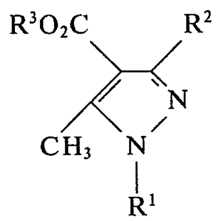

dans laquelle

$R^1$ représente hydrogène, formyle, acétyle, propionyle, chloracétyle, dichloracétyle, méthoxyacétyle, méthoxycarbonyle, ou phénoxycarbonyle éventuellement substitué une ou plusieurs fois par un halogène,
$R^2$ représente phényle, qui est substitué éventuellement une ou plusieurs fois par fluor, chlore, brome, méthyle, méthoxy, cyano, nitro, trifluorométhyle, trifluorométhoxy ou méthoxycarbonyle, ou un noyau aromatique hétérocyclique penta- ou hexagonal, éventuellement substitué par méthyle ou chlore, et contenant un ou deux hétéroatomes X, X signifiant, indépendamment l'un de l'autre, oxygène, soufre ou azote, ou représentant $\alpha$-naphtyle ou $\beta$-naphtyle,
$R^3$ représente méthyle, éthyle ou isopropyle ou un sel du dérivé pyrazolique.

7. Dérivé pyrazolique selon la revendication 6, choisi dans le groupe constitué de:

3-phényl-4-méthoxycarbonyl-5-méthyl-pyrazole,
3-phényl-4-éthoxycarbonyl-5-méthyl-pyrazole,
1-acétyl-3-m-nitrophényl-4-méthoxycarbonyl-5-méthyl-pyrazole,
3-m-méthylphényl-4-méthoxycarbonyl-5-méthyl-pyrazole,
3-m-fluorophényl-4-méthoxycarbonyl-5-méthyl-pyrazole,
3-o-fluorophényl-4-méthoxycarbonyl-5-méthyl-pyrazole,
1-acétyl-3-m-chlorophényl-4-méthoxycarbonyl-5-méthyl-pyrazole,
1-acétyl-3-m-bromophényl-4-méthoxycarbonyl-5-méthyl-pyrazole,
1-acétyl-3-(3,5-dichlorophényl)-4-méthoxycarbonyl-5-méthyl-pyrazole et
1-acétyl-3-(thiényl-2)-4-méthoxycarbonyl-5-méthylpyrazole.

**Revendications pour l'Etat Contractant: AT**

1. Herbicide contenant un dérivé pyrazolique de formule:

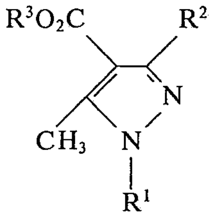

dans laquelle

$R^1$ représente hydrogène, formyle, acétyle, propionyle, chloracétyle, dichloracétyle, méthoxyacétyle, méthoxycarbonyle ou phénoxycarbonyle éventuellement substitué une ou plusieurs fois par un halogène,

$R^2$ représente phényle, qui est substitué éventuellement une ou plusieurs fois par fluor, chlore, brome, méthyle, méthoxy, cyano, nitro, trifluorométhyle, trifluorométhoxy ou méthoxycarbonyle, ou un noyau aromatique hétérocyclique penta- ou hexagonal, éventuellement substitué par méthyle ou chlore, et contenant un ou deux hétéroatomes X, X signifiant, indépendamment l'un de l'autre, oxygène, soufre ou azote, ou représentant $\alpha$-naphtyle ou $\beta$-naphtyle,

$R^3$ représente méthyle, éthyle ou isopropyle ou un sel du dérivé pyrazolique.

2. Herbicide contenant un véhicule solide ou liquide et un dérivé pyrazolique tel que défini à la revendication 1.

3. Procédé de préparation d'un herbicide, caractérisé par le fait qu'on mélange un véhicule solide ou liquide avec un dérivé pyrazolique tel que défini à la revendication 1.

4. Procédé de lutte contre la croissance indésirable de plantes, caractérisé par le fait qu'on traite le sol ou les plantes avec un dérivé pyrazolique tel que défini à la revendication 1.

5. Herbicide selon la revendication 1, contenant un dérivé pyrazolique choisi dans le groupe constitué de:

3-phényl-4-méthoxycarbonyl-5-méthyl-pyrazole,
3-phényl-4-éthoxycarbonyl-5-méthyl-pyrazole,
1-acétyl-3-m-nitrophényl-4-méthoxycarbonyl-5-méthyl-pyrazole,
3-m-méthylphényl-4-méthoxycarbonyl-5-méthyl-pyrazole,
3-m-fluorophényl-4-méthoxycarbonyl-5-méthyl-pyrazole,
3-o-fluorophényl-4-méthoxycarbonyl-5-méthyl-pyrazole,
1-acétyl-3-m-chlorophényl-4-méthoxycarbonyl-5-méthyl-pyrazole,
1-acétyl-3-m-bromophényl-4-méthoxycarbonyl-5-méthyl-pyrazole,
1-acétyl-3-(3,5-dichlorophényl)-4-méthoxycarbonyl-5-méthyl-pyrazole et
1-acétyl-3-(thiényl-2)-4-méthoxycarbonyl-5-méthyl-pyrazole.